# EUROPEAN PATENT APPLICATION

(11) **EP 4 506 354 A1**
(43) Date of publication of application: **12.02.2025**
(21) Application number: 23773967.7
(22) Date of filing: 23.03.2023
(51) Int. Cl.: C07J 5/00, C07J 9/00, C07J 21/00

(54) **7-KETOLITHOCHOLIC ACID INTERMEDIATE, SYNTHESIS METHOD THEREFOR, AND APPLICATION THEREOF**

(30) Priority: 25.03.2022 CN 202210321891; 07.06.2022 CN 202210641979
(71) Applicant: Suzhou Entech New-Material Technology Co., Ltd, Suzhou, Jiangsu 215004 (CN)
(72) Inventor: YANG, Yingquan, Suzhou, Jiangsu 215004 (CN); YUAN, Haitao, Suzhou, Jiangsu 215004 (CN); ZHOU, Panlong, Suzhou, Jiangsu 215004 (CN); WANG, Fei, Suzhou, Jiangsu 215004 (CN); WU, Hucheng, Suzhou, Jiangsu 215004 (CN)
(74) Representative: Gille Hrabal Partnerschaftsgesellschaft mbB Patentanwälte
(86) International application number: PCT/CN2023/083436
(87) International publication number: WO 2023/179721

(57) **Abstract**

The present invention provides a synthesis method for a 7-ketolithocholic acid or an intermediate thereof, which is prepared from a new intermediate **I-1.** According to the method, phytosterol degradation product bisnoralcohol is used as a starting material, and the 7-ketolithocholic acid or the intermediate thereof is obtained by means of an oxidation reaction, a Knoevenagel reaction (or wittig reaction), hydrogenation, a ketal protection reaction, an allylic oxidation reaction, ketal removal protection, and the hydrogenation. According to the method of the present invention, raw materials are easily available, the yield is high, reaction conditions are simple and mild, and the method is suitable for industrial production.

## Description

The present invention claims the priorities of the prior application with Patent Application No. 202210321891.5, entitled "Method for Synthesizing 7-Ketolithocholic Acid Intermediate and Use thereof" filed on March 25, 2022 to China National Intellectual Property Administration and the prior application with Patent Application No. 202210641979.5, entitled "7-Ketolithocholic Acid Intermediate and Synthesis Method therefor and Use thereof" filed on June 7, 2022 to China National Intellectual Property Administration, the contents of which are incorporated by reference herein in their entirety as part of the present invention.

### TECHNICAL FIELD

The present invention belongs to the field of pharmaceutical synthesis and specifically relates to a 7-ketolithocholic acid intermediate and a synthesis method therefor and use thereof.

### BACKGROUND

7-ketolithocholic acid has the CAS number of 4651-67-6, the molecular formula of C₂₄H₃₈O₄, the molecular weight of 390.56, and the structural formula shown below:

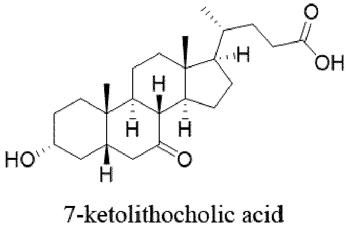

7-ketolithocholic acid is an important drug intermediate. 7-ketolithocholic acid may be used as an intermediate to synthesize chenodeoxycholic acid (Tetrahedron Letters Volume 24, Issue 24, 1983, Pages 2487-2490), ursodeoxycholic acid (J. Org. Chem. 1993, 58, 499-501), obeticholic acid (J. Med. Chem. 2002, 45, 17, 3569-3572) and the like.

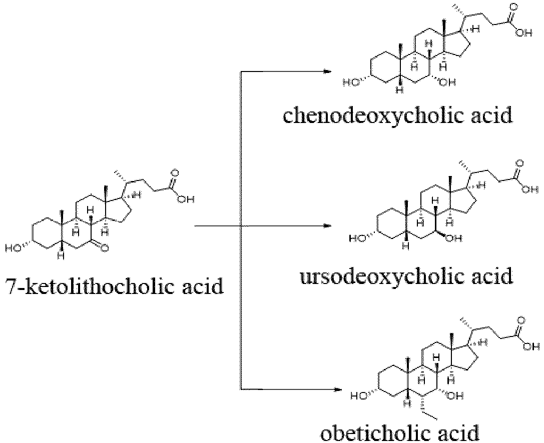

At present, most of the production processes for ursodeoxycholic acid and obeticholic acid are basically based on a method using 7-ketolithocholic acid as a raw material. Therefore, it becomes very important to effectively obtain 7-ketolithocholic acid.

From the current literatures and patent researches, 7-ketolithocholic acid is mainly synthesized by the following methods.

### I. Using cholic acid as the raw material

WO2014020024A1 reports that cholic acid is used as a raw material, the carboxy group on the side chain is esterified by methanol and hydrochloric acid, hydroxyl groups at both 3-position and 7-position are protected by acetic anhydride, hydroxyl at 12-position is oxidized by sodium hypochlorite to a carbonyl, ketone at 12-position is subjected to Wolff-Kishner-Huang-Minlon reduction, and finally, hydroxyl at 7-position is selectively oxidized by sodium hypobromite to ketone so as to obtain a target compound, namely 7-ketolithocholic acid. During the whole process, it is required to use the Wolff-Kishner-Huang-Minlon reaction performed at very high temperature. The reaction is performed at a relatively high temperature and hydrazine hydrate is highly toxic and explosive. Therefore, the requirements for equipment are relatively high.

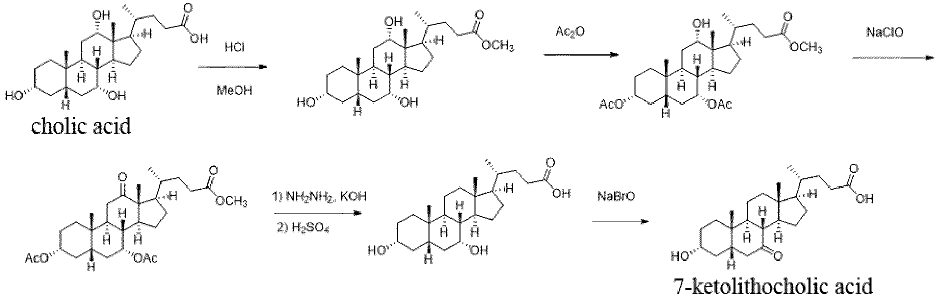

### II. Using chenodeoxycholic acid as the raw material

CN106046095 reports a method for obtaining 7-ketolithocholic acid by NBS oxidation of chenodeoxycholic acid in acetone and water. However, the chenodeoxycholic acid is expensive and use thereof is limited.

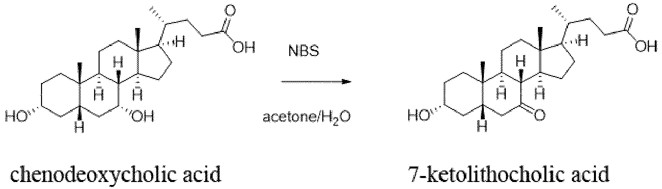

### III. Using hyocholic acid as the raw material

A patent publication CN110423261A reports a preparation method using hyocholic acid as a raw material. The disadvantages of the scheme are that: 1) chromium reagents such as Jones reagent are used, resulting in more pollution and environmental protection pressure; 2) a part of the reagents such as lithium iodide and TBSCl are expensive, and the whole cost of the route is high; and 3) pyridine is used as a solvent in 3^{rd} step, which has a strong odor and high toxicity.

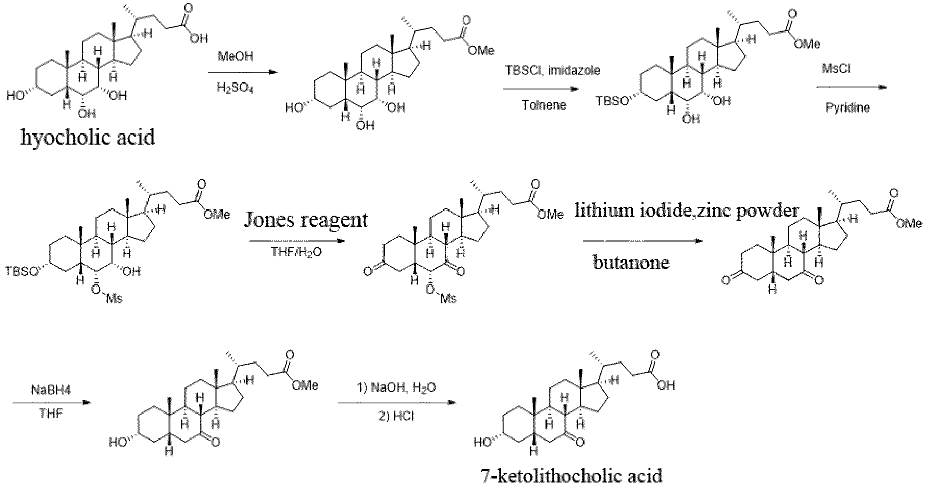

In addition to various disadvantages of the three methods mentioned above, another disadvantage is that the methods use animal-derived cholic acid. The animal body generally contains many animal viruses such as swine fever virus, avian influenza virus, prion and other various physiologically active substances, which have a certain biological toxicity to human body. Therefore, there is a need to find a new, safe and effective 7-ketolithocholic acid intermediate and a synthesis method therefor.

### SUMMARY

In order to solve the above technical problems, the present invention aims to provide a method for preparing 7-ketolithocholic acid or an intermediate thereof. The preparation method uses a new intermediate I-1, which is simple, has a high yield and is safe and effective. Another objective of the present invention is to provide a new intermediate I-1 and a preparation method therefor.

The purposes of the present invention are realized by the following technical solutions.

The present invention provides a method for preparing a compound of formula I, comprising subjecting a compound I-1 to a hydrogenation reaction under the presence of a catalyst to obtain the compound of formula I, 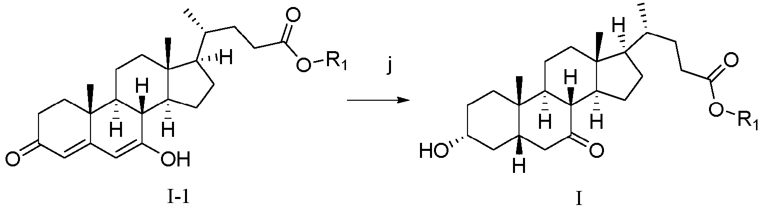 wherein R₁ is H or alkyl, for example, C₁₋₆ alkyl, such as methyl, ethyl, n-propyl or tert-butyl.

According to an embodiment of the present invention, in the reaction, the catalyst is selected from a Raney Ni catalyst, a Pd/C catalyst, a Pt/C catalyst or a Ru/C catalyst.

According to an embodiment of the present invention, in the reaction, the mass ratio of the compound I-1 to the catalyst is (2-20):1, for example (5-15):1, exemplarily 10:1.

According to an embodiment of the present invention, in the reaction, the reaction is performed in the presence of a solvent, wherein the solvent is an amide solvent, for example, the solvent is selected from at least one of N,N-dimethylformamide, N,N-dimethylacetamide, formamide, N-methylpyrrolidone, N-methylformamide, N-methylacetamide and N,N-dimethylpropyleneurea.

According to the present invention, in the above method, when R₁ in the compound of formula I is alkyl, the method further comprises: hydrolyzing the compound of formula I in which R₁ is alkyl to obtain the compound of formula I in which R₁ is H, namely 7-ketolithocholic acid,

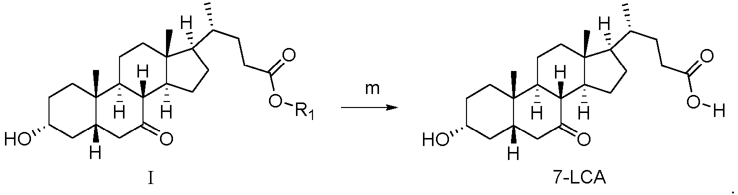

According to the present invention, the hydrolysis reaction is performed under an alkaline condition, for example in the presence of sodium hydroxide, potassium hydroxide, etc.

The present invention further provides an intermediate compound I-1 or I-2 shown below: 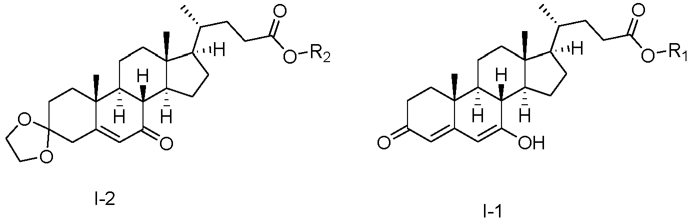 wherein R₁ is as defined above and R₂ is alkyl.

The present invention further provides a method for preparing the compound I-1, comprising: 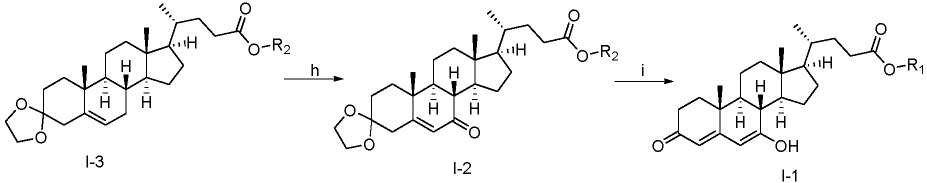 wherein R₁ is as defined above and R₂ is alkyl;
h) subjecting the compound I-3 to an allylic oxidation reaction to obtain the compound I-2; and
i) subjecting the compound I-2 to a de-protection of ethylene glycol to obtain the compound I-1 with R₁ as alkyl; or subjecting the compound I-2 to a de-protection of ethylene glycol and a hydrolysis reaction to obtain the compound I-1 with R₁ as H.

According to an embodiment of the present invention, in step h), in the presence of an oxidant and a catalyst, a compound I-3 is subjected to an allylic oxidation reaction to obtain the compound I-2.

According to an embodiment of the present invention, step h) is performed under the following reaction system of h1 or h2:
h1: the oxidant is oxygen or air, and the catalyst is a reaction system of N-hydroxyphthalimide (NHPI) and cobalt acetate; and a free radical initiator, for example, benzoyl peroxide, may further be added to the above system; and according to an embodiment of the present invention, the molar ratio of the compound I-3, the catalyst, the initiator and the cobalt acetate is (1-20):1:1:(0.01-0.5), for example, (5-15):1:1:(0.02-0.1), exemplarily 10:1:1:0.05; and
h2: the oxidant is tert-butyl hydroperoxide (TBHP) and the catalyst is a reaction system of manganese (III) acetate, manganese (III) acetate dihydrate, or cuprous iodide (CuI); and according to an embodiment of the present invention, the molar ratio of the compound I-3, the oxidant, and the catalyst is 1:(1-20):(0.5-10), for example, 1:(2-10):(1-5), exemplarily 1:5:2.74.

According to an embodiment of the present invention, in step i), the de-protection of ethylene glycol is performed in the presence of an acid, wherein the acid is for example at least one of concentrated sulfuric acid, p-toluenesulfonic acid and hydrochloric acid. The hydrolysis reaction is performed under an alkaline condition, for example in the presence of sodium hydroxide and/or potassium hydroxide.

According to an embodiment of the present invention, a method for preparing the compound I-3 comprises the following Method I or Method II:

### Method I comprises:

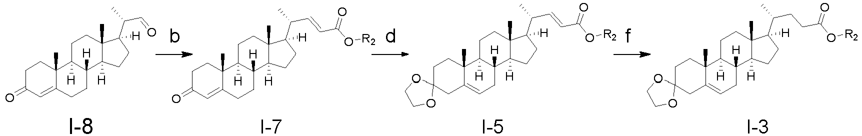

wherein R₂ is as defined above;
b) subjecting a compound I-8 and monoalkyl malonate ( 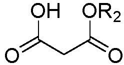 ) to a Knoevenagel condensation to obtain a compound I-7; or subjecting the compound I-8 and 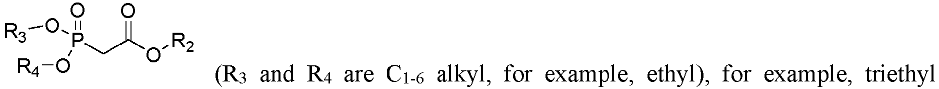 phosphonoacetate ( 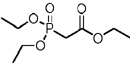 ) to a wittig reaction to obtain the compound I-7;
d) subjecting the compound I-7 to a protection of ethylene glycol to obtain a compound I-5; and
f) subjecting the compound I-5 to a reduction reaction to obtain the compound I-3.

According to an embodiment of the present invention, the molar ratio of the compound I-8 to the monoalkyl malonate in step b) is 1:(0.5-10), for example, 1:(1-5), exemplarily 1:1.5.

According to an embodiment of the present invention, step b) is performed in the presence of a catalyst, wherein the catalyst is DMAP.

According to embodiment of the present invention, step d) is performed in the presence of a catalyst, wherein the catalyst is catalyst A and/or catalyst B, wherein the catalyst A is selected from p-toluenesulfonic acid or concentrated sulfuric acid, and the catalyst B is selected from trimethyl orthoformate, triethyl orthoformate or trimethyl orthoacetate. In one embodiment, when the catalyst is the catalyst A and the catalyst B, the mass ratio of the catalyst A to the catalyst B may be 1:(0-100), for example, 1:(40-80), exemplarily 1:56.

According to an embodiment of the present invention, in step d), the mass ratio of the compound I-7, the ethylene glycol and the catalyst is 1:(0.2-5):(0.1-5), for example, 1:(0.5-3):(0.2-2), exemplarily 1:1:0.57.

According to an embodiment of the present invention, step f) is performed in the presence of a catalyst, wherein the catalyst is Pd/C.

According to an embodiment of the present invention, in step f), the mass ratio of the compound I-5 to the catalyst is (2-30):1, for example (10-25):1, exemplarily 20:1.

### Method II comprises:

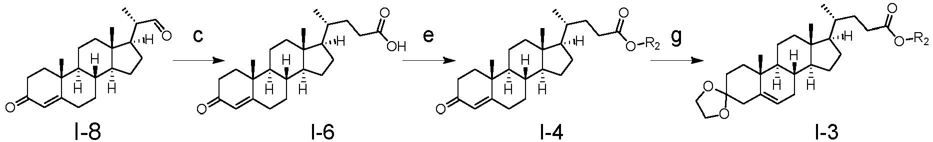

wherein R₂ is as defined above;
c) subjecting the compound I-8 and a Meldrum's acid compound ( 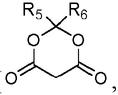 wherein R₅ and R₆ are C₁₋₆ alkyl (for example, methyl), or R₅ and R₆ together with the carbon atom to which they are connected form a C₃₋₈ cycloalkyl) to a Knoevenagel condensation to obtain a compound I-6;
e) reacting the compound I-6 with R₂OH to obtain a compound I-4; and
g) subjecting the compound I-4 to a protection of ethylene glycol to obtain a compound I-3.

According to an embodiment of the present invention, in step c), the molar ratio of the compound I-8 to the Meldrum's acid compound is 1:(0.1-10), for example, 1:(0.5-5), exemplarily 1:1.2.

According to an embodiment of the present invention, step e) is performed in the presence of a catalyst, wherein the catalyst is selected from at least one of concentrated sulfuric acid, p-toluenesulfonic acid and hydrochloric acid.

According to an embodiment of the present invention, step g) is performed in the presence of a catalyst, wherein the catalyst is catalyst A and/or catalyst B, wherein the catalyst A is selected from p-toluenesulfonic acid or concentrated sulfuric acid, and the catalyst B is selected from trimethyl orthoformate, triethyl orthoformate or trimethyl orthoacetate. In one embodiment, when the catalyst is the catalyst A and the catalyst B, the molar ratio of the catalyst A to the catalyst B may be 1:(0-100), for example, 1:(20-80), exemplarily 1:0, 1:29 or 1:73.

According to an embodiment of the present invention, in step g), the molar ratio of the compound I-4, the ethylene glycol and the catalyst is 1:(1-50):(1-20), for example, 1:(5-25):(1-10), exemplarily 1:6.5:1.5 or 1:21.6:6.2.

According to an embodiment of the present invention, the method for preparing the compound I-8 comprises the following step: subjecting a compound BA to an oxidation reaction to obtain the compound I-8,

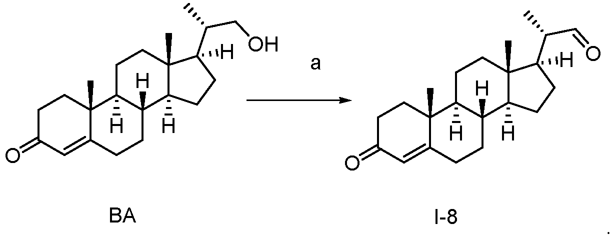

### Beneficial effects

The present invention provides a new method for synthesizing 7-ketolithocholic acid or an intermediate thereof, which is prepared from a new intermediate I-1. Since the new intermediate I-1 is used for reaction, the generation of the compound of formula I can be more favored with fewer isomers and higher yields. In addition, an amide solvent having a weak basicity is used in the reaction, favoring the conversion of a tautomer towards the compound of formula I. The preparation method of the present invention has the advantages of mild and simple reaction conditions, high yield and suitability for industrial production.

According to the present invention, phytosterol biodegradable product bisnoralcohol (BA) as a starting material can be converted to 7-ketolithocholic acid through a oxidation reaction, a Knoevenagel condensation (or a wittig reaction), a hydrogenation reaction, an esterification reaction, a ketal protection, an allylic oxidation reaction, a ketal de-protection, a hydrogenation reaction and a hydrolysis reaction. The method has the advantages of easily available raw materials, high yield, simple and mild reaction conditions and suitability for industrial production.

### Definition and description of terms

Unless otherwise indicated, the definitions of groups and terms described in the description and claims of the present application, including definitions thereof as examples, exemplary definitions, preferred definitions, definitions described in tables, definitions of specific compounds in the examples, etc., may be combined with each other at will. The definitions of the groups after such combination and structures of compounds should be understood to be within the scope described in the description and/or claims of the present application.

The term "C₁₋₆ alkyl" represents a straight or branched saturated monovalent hydrocarbyl having 1, 2, 3, 4, 5 or 6 carbon atoms. The alkyl is, for example, methyl, ethyl, n-propyl, n-butyl, n-pentyl, n-hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, etc. or isomers thereof.

The term "C₃₋₈ cycloalkyl" should be understood to mean a saturated, monovalent and monocyclic hydrocarbon ring having 3, 4, 5, 6, 7, or 8 carbon atoms, for example, cyclopropyl, cyclobutyl, cyclopentyl, cyclohexyl, cycloheptyl or cyclooctyl.

### DETAILED DESCRIPTION

The technical solutions of the present invention will be further described in detail below in conjunction with specific examples. It should be understood that the following examples only exemplarily illustrate and explain the present invention and should not be construed as limiting the protection scope of the present invention. All the techniques realized on the basis of the above-mentioned contents of the present invention are covered in the protection scope of the present invention.

Unless otherwise indicated, the raw materials and reagents used in the following examples are all commercially-available, or may be prepared by known methods.

### Example 1 Synthesis of A8

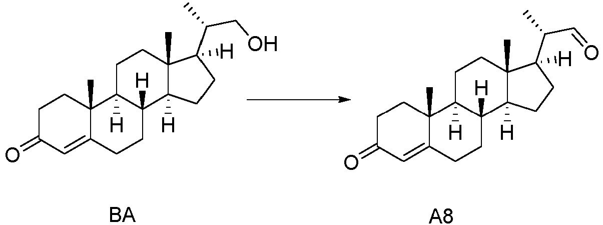

NaBr (3.2 g, 31.06 mmol), TEMPO (1 g, 6.4 mmol) and NaHCO₃(30.6 g, 364 mmol) were added to a mixture of DCM (600 mL) and water (100 mL) containing BA (100 g, 303 mmol). The reaction solution was cooled to 0-5 °C and 10% NaClO solution (115 g, 155 mmol) was dropwise added while stirring. After the dropwise addition is completed, stirring was continued for 30 min. The reaction was quenched with 5 g of Na₂SO₃ and then stirred for 30 min. The reaction solution was stood and subjected to a liquid separation. The lower layer was washed once with saturated NaHCO₃, dried and concentrated under reduced pressure to obtain 90 g of a white solid A8 with the yield of 90.5% and the purity of 90% by HPLC.

### Example 2 Synthesis of A8

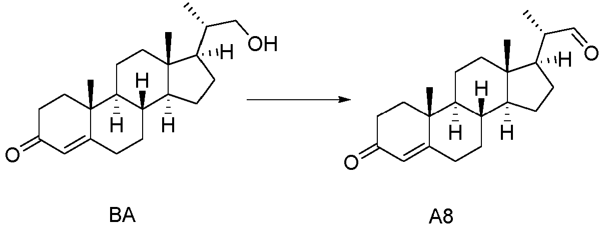

After BA (33 g, 100 mmol) and DCM (100 mL) were added to a reaction flask and dissolved with stirring, hydrochloric acid (3.7%, 2 mmol, 2 g), TEMPO (0.31 g, 2 mmol) and sodium bromate (5 g, 35 mmol) were added. The mixture was reacted at 25 °C for 16 h while stirring, then water (100 mL) was added, and the mixture was subjected to a liquid separation. The organic phase was washed with an aqueous saturated sodium bicarbonate solution (100 mL) and a saturated sodium chloride solution (100 mL), dried over anhydrous sodium sulfate, filtered, and concentrated to remove the solvent. The residue was subjected to column chromatography on silica gel (n-hexane: EtOAc = 10:1) to obtain 28.2 g of A8 with the yield of 86% and the purity of 96% by HPLC.

### Example 3 Synthesis of A7

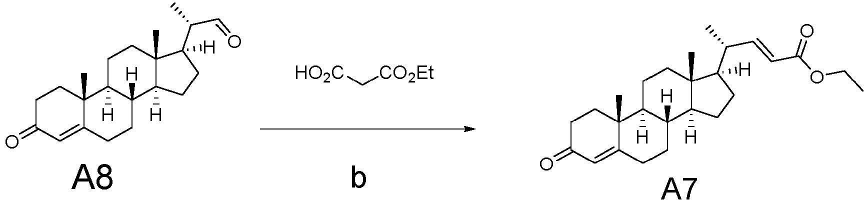

A8 (65.6 g, 200.00 mmol) prepared in Example 2 and monoethyl malonate (39.6 g, 300.00 mmol) were added to a 1 L three-necked flask containing 500 mL of DMF, stirred well, and DMAP (2.44 g, 20 mmol) was added at 25 °C and the reaction was stirred for 16 h. When TLC monitored that the reaction of A8 was complete, 500 mL of methyl tert-butyl ether was added. The organic phase was successively washed with aqueous saturated NaHCO₃ solution (500 mL) and a saturated sodium chloride solution (500 mL), dried over anhydrous sodium sulfate, filtered, and concentrated under reduced pressure to obtain 90 g of a white solid A7 with the yield of 90.5% and the purity of 98% by HPLC.

### Example 4 Synthesis of A7

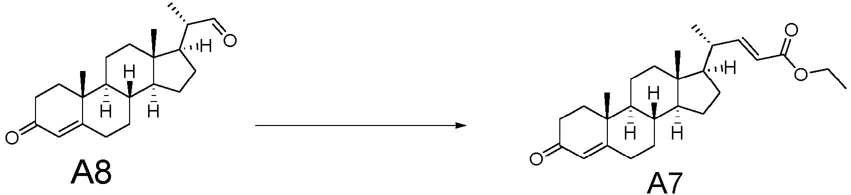

40.9 g of triethyl phosphonoacetate and 400 mL of THF were added to a 1 L three-necked flask. The temperature was controlled to 0-5 °C, 6.7 g of 60% NaH was added in batches with stirring, and a large amount of gas was released. After stirring for 1 h, 300 mL of the previously prepared THF solution containing 49.8 g of A8 prepared in Example 2 was dropwise added to the above system using a 500 mL pressure equalizing funnel. After the dropwise addition was completed, the stirring was continued for 2 h. The reaction was quenched by adding 20 g of ammonium chloride in 200 mL of water. After stirring for 1 h, liquid separation was performed. The aqueous phase was extracted once with 200 mL of EA, and the organic phases were combined, dried and concentrated under reduced pressure to obtain 55 g of a crude product. The crude product was slurried with 80 mL of petroleum ether and filtered to obtain 55 g of a off-white solid A7 with the yield of 91% and the purity of 95% by HPLC.

### Example 5 Synthesis of A5

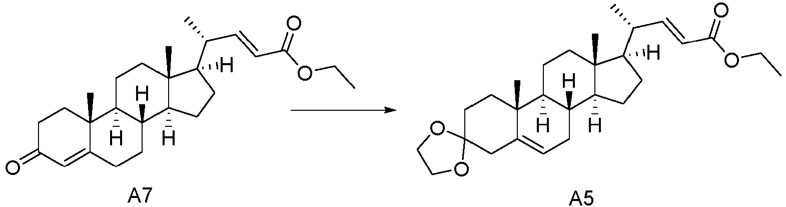

50 g of A7 prepared in Example 3, 50 g of ethylene glycol, 600 mL of DCM, 28 g of triethyl orthoformate and 0.5 g of p-toluenesulfonic acid were added to a 1 L three-necked flask under protection of N₂, and stirred at 25 °C for 10 h. When TLC showed the reaction was complete, 1 mL of triethylamine was added and stirred for 30 min. The reaction solution was washed with 100 mL of water, subjected to liquid separation, dried and concentrated to obtain 55 g of an A5 crude product. The crude product was refluxed and slurried with 150 mL of petroleum ether to obtain 48.5 g of A5 with the yield of 87.3% and the purity of 97% by HPLC.

### Example 6 Synthesis of A3

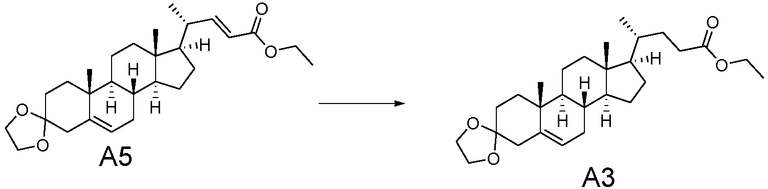

2.01 g of A5 prepared in Example 5 and 0.1 g of 5% palladium carbon were added to 20 mL of ethyl acetate, and stirred at 25 °C under a pressure of 0.2 MPa hydrogen for 1 h. The reaction solution was filtered and concentrated under reduced pressure to obtain 1.95 g of a white solid A3 with the yield of 97% and the purity of 97% by HPLC.

### Example 7 Synthesis of A6

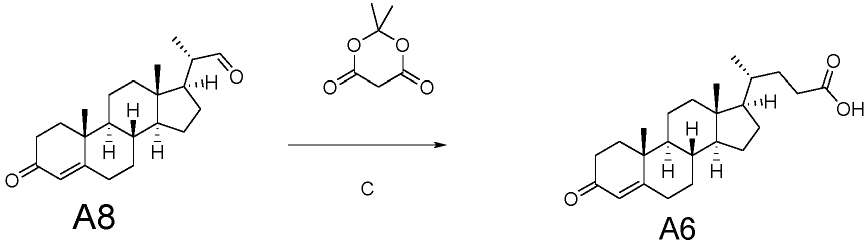

A8 (258 g, 789 mmol, 1.0 eq) prepared in Example 2, Meldrum's acid (136 g, 947 mmol, 1.2 eq) and a mixed solution of formic acid and triethylamine (formic acid/triethylamine = 5:2, 1,000 mL) were added to a reaction flask. The reaction mixture was heated at 110 °C for 16 h. When TLC monitored that A8 was reacted completely, the reaction product was poured into ice water (1,000 mL), then adjusted to pH 10 with 1 N NaOH, and washed with ethyl acetate (200 mL×2). The aqueous phase was adjusted to pH 2 with 1 N HCl, and extracted with ethyl acetate (200 mL×3). The organic phases were combined to obtain 288 g of a crude product which was refined with acetone to obtain 270 g of the product with the yield of 91.8% and the purity of 96% by HPLC.

### Example 8 Synthesis of A4

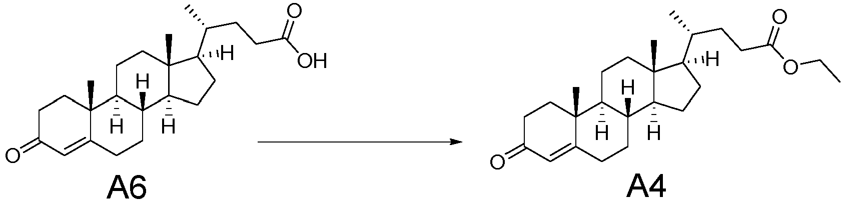

A6 prepared in Example 7 (100 g, 270 mmol), ethanol (300 mL) and hydrochloric acid (20 mL, 37.5%) were added to a reaction flask and stirred at 25 °C for 16 h. When TLC monitored that A6 was reacted completely, the reaction product was concentrated under reduced pressure to remove excess ethanol and diluted with ethyl acetate (300 mL). The organic phase was successively washed with water (300 mL), an aqueous saturated sodium bicarbonate solution (300 mL) and an aqueous saturated sodium chloride solution (300 mL), dried over anhydrous sodium sulfate, filtered and concentrated to obtain 100 g of A4 with the yield of 93% and the purity of 96% by HPLC. ESI-MS[M+H]⁺ 445.3 3.

### Example 9 Synthesis of A4

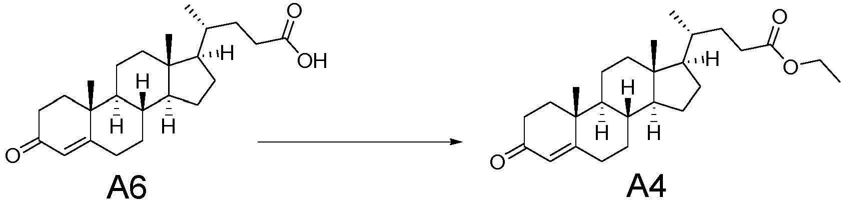

A6 prepared in Example 7 (74.4 g, 200 mmol), ethanol (200 mL) and concentrated sulfuric acid (5 mL) were added to a reaction flask and reacted for 24 h under refluxing and stirring. When TLC monitored that A6 was reacted completely, the reaction product was concentrated under reduced pressure to remove excess ethanol, and diluted with dichloromethane (200 mL). The organic phase was successively washed with water (200 mL), an aqueous saturated sodium bicarbonate solution (200 mL) and an aqueous saturated sodium chloride solution (200 mL), dried over anhydrous sodium sulfate, filtered and concentrated to obtain 73.6 g of A4 with the yield of 92% and the purity of 97% by HPLC. ESI-MS[M+H]⁺445.33.

### Example 10 Synthesis of A3

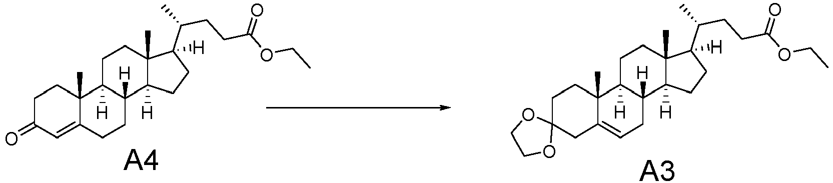

A4 (50 g, 125 mmol) prepared in Example 9, ethylene glycol (50 g, 806 mmol), DCM (600 mL), triethyl orthoformate (28 g, 189 mmol) and p-toluenesulfonic acid (0.5 g, 2.6 mmol) were added to a 1 L three-necked flask under protection of N₂, and stirred at 25 °C for 10 h. When TLC showed the reaction was completed, 1 mL of triethylamine was added and stirred for 30 min. The reaction solution was washed with 100 mL of water and subjected to liquid separation. The organic phase was dried over anhydrous sodium sulfate and concentrated to obtain 58 g of an A3 crude product. The crude product was refluxed and slurried with 150 mL of petroleum ether to obtain 51.8 g of A3 with the yield of 95% and the purity of 95% by HPLC. ESI-MS[M+H]⁺ 444.35. ¹H-NMR(CDCl₃, 400MHz) δ(ppm):5.36-5.34(m, 1H), 4.12(q, J=7.2Hz, 2H), 3.98-3.91(m, 4H), 2.58-2.54(m, 1H), 2.38-2.30(m, 1H), 2.13-2.09(m, 1H), 2.00-1.92(m, 2H), 1.89-1.73(m, 4H), 1.67-1.60(m, 3H), 1.49-1.40(m, 4H), 1.36-1.30(m, 2H), 1.25(t, J=7.2Hz, 3H), 1.20-1.06(m, 4H), 1.02(s, 3H), 0.92(d, J=6.4Hz, 3H), 0.68(s, 3H).

### Example 11 Synthesis of A3

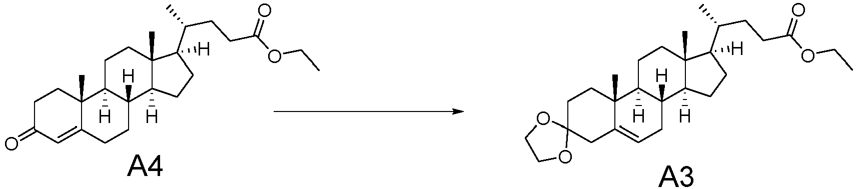

A4 (50 g, 125 mmol) prepared in Example 9, ethylene glycol (150 mL, 2,702 mmol), 90 g of trimethyl orthoacetate (90 g, 750 mmol) and p-toluenesulfonic acid (5 g, 26 mmol) were added to a reaction flask under protection of inert gas, and reacted at 25 °C for 2 h to precipitate a large amount of a solid. Methanol (100 mL) was added to disperse, the pH was adjusted to 7-8 with triethylamine, and the reaction solution was filtered and dried to obtain 50.2 g of a compound A3 with the yield of 92% and the purity of 98% by HPLC. ESI-MS[M+H]⁺ 444.35.

### Example 12 Synthesis of A3

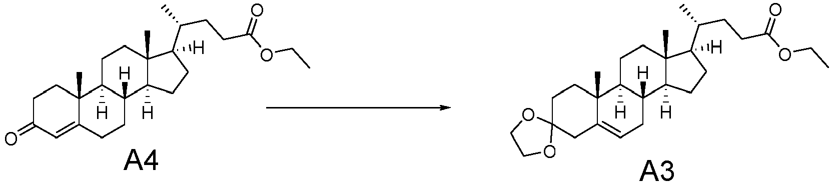

A4 (50 g, 125 mmol) prepared in Example 9, ethylene glycol (39 g, 625 mmol), p-toluenesulfonic acid (5 g, 26 mmol) and toluene (200 mL) were added in a reaction flask under protection of inert gas, and the reaction mixture was reacted for 24 h while heating, refluxing and separating water. The reaction mixture was cooled to 25 °C and successively washed with water (200 mL), an aqueous saturated sodium bicarbonate solution (200 mL) and an aqueous saturated sodium chloride solution (200 mL), dried over anhydrous sodium sulfate and concentrated to obtain 57 g of an A3 crude product. The crude product was refluxed and slurried with 150 mL of petroleum ether to obtain 46.3 g of A3 with the yield of 85% and the purity of 90% by HPLC. ESI-MS[M+H]⁺ 444.35.

### Example 13 Synthesis of A2

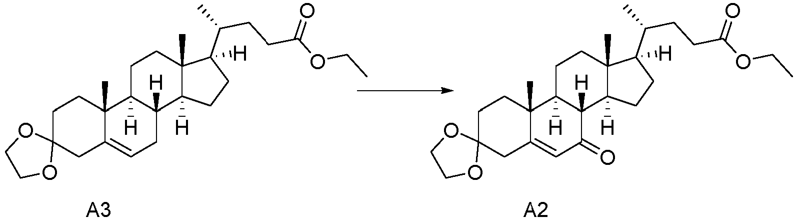

The compound A3 prepared in Example 11 (44.4 g, 100 mmol), N-hydroxyphthalimide (NHPI) (1.63 g, 10 mmol), benzoyl peroxide (2.4 g, 10 mmol), cobalt acetate (88 mg, 0.5 mmol) and cyclohexanone (200 mL) were added to a reaction flask. The reaction mixture was stirred at 25 °C for 10 min, heated to 60 °C, and reacted while blowing air. After the reaction was completed, the reaction solution was concentrated to remove cyclohexanone, then dichloromethane was added, and the reaction solution was filtered to remove the catalyst. Triethylamine (10 g, 100 mmol) and acetic anhydride (10 g, 100 mmol) were added, and the mixture was reacted at 25 °C for 10 h. After the reaction was complete, the reaction solution was quenched with ethanol, and concentrated. After ethanol was added, the product was obtained and dried to give 30 g of a compound A2 with the yield of 65.8% and the purity of 95% by HPLC. ESI-MS[M+H]⁺ 459.35. ¹H-NMR(CDCl₃, 400MHz) δ(ppm):5.67(s, 1H), 4.12(brs, 2H), 3.97(brs, 4H), 2.70-2.66(m, 1H), 2.45-2.22(m, 5H), 2.00-1.75(m, 6H), 1.59-1.11(m, 17H), 0.94(s, 3H), 0.69(s, 3H).

### Example 14 Synthesis of A2

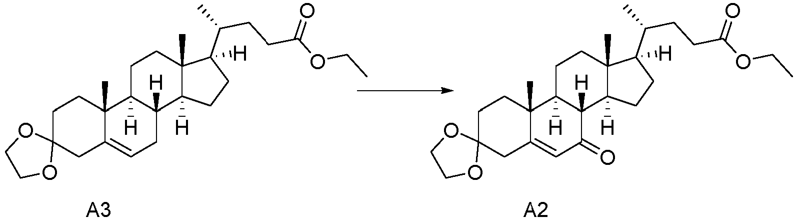

A3 (100 g, 270 mmol) prepared in Example 11, ethyl acetate (200 mL), TBHP (270 mL, 1,350 mmol, 5.0 M n-decane solution), 3A molecular sieve (20 g) and manganese (III) acetate dihydrate (740 mg, 27 mmol) were added to a reaction flask and reacted at 25 °C for 48 h. The reaction system was diluted with methyl tert-butyl ether (200 mL) and filtered through celite. The filtrate was concentrated under reduced pressure to remove the solvent and purified by column chromatography (n-hexane:EtOAc = 5:1) to obtain 110 g of a product with the yield of 90% and the purity of 98% by HPLC. ESI-MS[M+H]⁺ 459.35.

### Example 15 Synthesis of A2

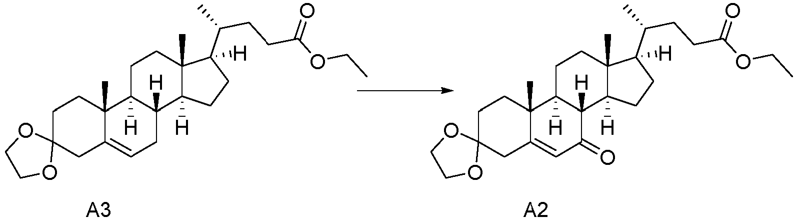

A3 (44 g, 100 mmol) prepared in Example 11, TBHP (120 mL, 600 mmol, 5.0 M n-decane solution), cuprous iodide (0.19 g, 1 mmol) and acetonitrile (200 mL) were added to a reaction flask, and reacted at 50 °C for 20 h. The reaction system was filtered through celite. The filtrate was concentrated under reduced pressure to remove the solvent and purified by column chromatography (n-hexane:EtOAc = 5:1) to obtain 38 g of a product with the yield of 83% and the purity of 96% by HPLC. ESI-MS[M+H]⁺ 459.35.

### Example 16 Synthesis of A1

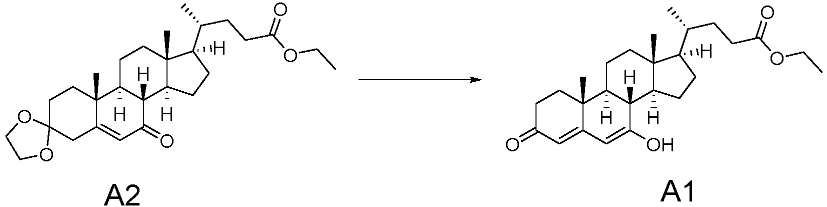

Water (40 mL) and THF (100 mL) were added to a 1 L single-neck flask, concentrated sulfuric acid (4 g, 40.8 mmol) was added while stirring at 0 °C, stirring was performed for 10 min, then A2 (12 g, 26.2 mmol) prepared in Example 14 was added, the cold bath was removed, and stirring was performed overnight at 25 °C. After adding water (100 mL), NaHCO₃ (10 g, 119 mmol) was added in batches to control the pH of 7-8. Then the reaction system was extracted twice with 200 mL of EtOAc. The organic phases were combined and concentrated under reduced pressure to obtain a crude product as a yellow oil. The crude product was purified by column chromatography (n-hexane:EtOAc = 3:1) to obtain 9.97 g of a solid A1 with the yield of 92% and the purity of 95% by HPLC. ESI-MS[M+H]⁺ 415.30.

### Example 17 Synthesis of A1

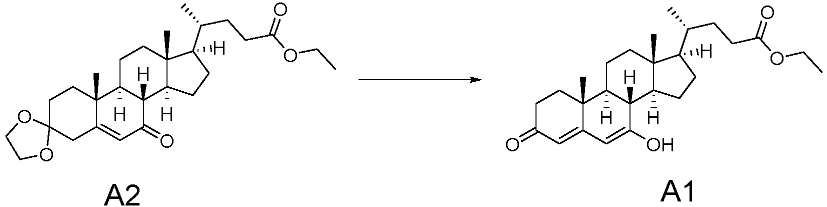

A2 prepared in Example 14 (54 g, 118 mmol) and p-toluenesulfonic acid monohydrate (5.2 g, 27.4 mmol) were added to a 1 L reaction flask containing 200 mL of toluene and 50 mL of water, stirred at 50 °C for 1 h, and cooled to 25 °C. The reaction was quenched by adding NaHCO₃ (10 g, 119 mmol). The reaction solution was stood for liquid separation and the aqueous phase was extracted once with toluene (100 mL). The organic phases were combined and concentrated under reduced pressure to obtain a crude product as a yellow oil. The crude product was purified by column chromatography (n-hexane:EtOAc = 3:1) to obtain 46.8 g of a light yellow solid A1 with the yield of 96% and the purity of 98% by HPLC. ESI-MS: [M+H]⁺ 415.30

¹H-NMR(DMSO-d6, 400MHz) δ(ppm):10.25(brs, 1H), 5.29-5.28(m, 2H), 4.03(q, J=7.2Hz, 2H), 2.41-2.12(m, 6H), 1.98-1.66(m, 4H), 1.11-1.53(m, 10H), 1.17(t, J=7.2Hz, 3H), 1.05(s, 3H), 0.91(d, J=6Hz, 3H), 0.66(s, 3H).

### Example 18 Synthesis of A

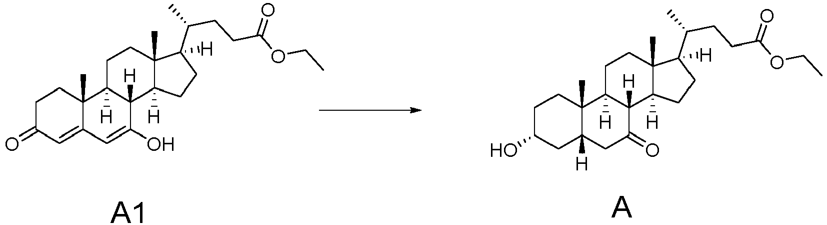

A1 (3.2 g, 7.73 mmol) prepared in Example 17 was dissolved in N,N-dimethylformamide (DMF) (32 mL) and then 0.32 g of Raney nickel was added. The reaction mixture was hydrogenated at 25 °C under 0.1 MPa hydrogen for 12 h. After the hydrogenation was completed, the reaction solution was filtered and concentrated under reduced pressure to obtain 3.3 g of a crude product A. The crude product was refined with acetone to obtain 2.84 g of a white solid A with the yield of 88% and the purity of 98% by HPLC. ESI-MS [M+H]⁺ 419.30.

¹H-NMR(DMSO-d6, 400MHz) δ(ppm): 4.48(d, J=4.8Hz, 1H), 4.04(q, J=6.8Hz, 2H), 2.90(dd, J=6Hz, 12Hz, 1H), 2.47-2.41(m, 1H), 2.36-2.27(m, 1H), 2.22-2.15(m, 1H), 2.09-2.02(m, 1H), 1.94-1.90(m, 1H), 1.85-1.76(m, 2H), 1.73-1.64(m, 4H), 1.50-1.45(m, 2H), 1.39-1.30(m, 4H), 1.27-1.21(m, 2H), 1.17(t, J=6.8Hz, 3H), 1.14(s, 3H), 1.11-1.01(m, 5H), 0.96-0.77(m, 2H), 0.88(d, J=6.4Hz, 3H), 0.61(s, 3H).

### Example 19 Synthesis of A

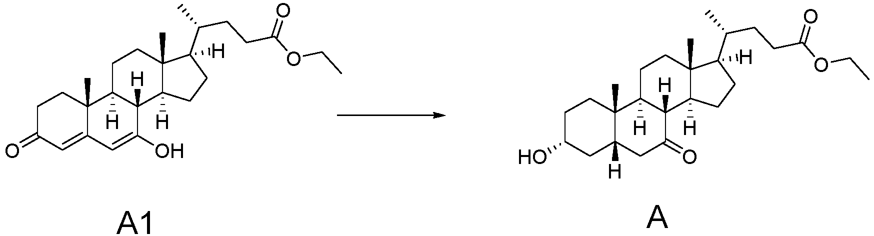

A1 (3.2 g, 7.73 mmol) prepared in Example 17 was dissolved in N,N-dimethylacetamide (32 mL) and then 0.32 g of Raney nickel was added. The reaction mixture was hydrogenated at 25 °C under 0.1 MPa hydrogen for 12 h. After the hydrogenation was completed, the reaction solution was filtered and concentrated under reduced pressure to obtain 3.4 g of a crude product A. The crude product was refined with acetone to obtain 2.74 g of a white solid A with the yield of 85% and the purity of 98% by HPLC. ESI-MS [M+H]⁺ 419.30.

### Example 20 Synthesis of A

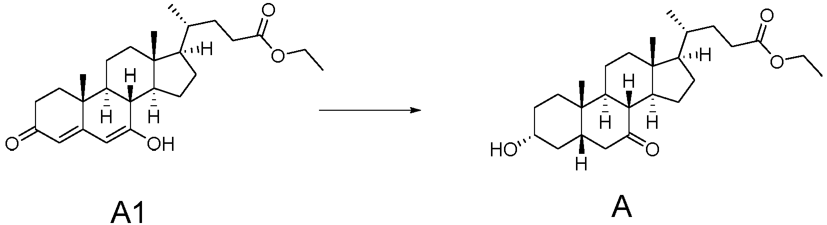

A1 (3.2 g, 7.73 mmol) prepared in Example 17 was dissolved in N-methylpyrrolidone (32 mL) and then 0.32 g of Raney nickel was added. The reaction mixture was hydrogenated at 25 °C under 0.1 MPa hydrogen for 12 h. After the hydrogenation was completed, the reaction solution was filtered and concentrated under reduced pressure to obtain 3.0 g of a crude product A. The crude product was refined with acetone to obtain 2.52 g of a white solid A with the yield of 78.3% and the purity of 95% by HPLC. ESI-MS: [M+H]⁺ 419.30.

### Example 21 Synthesis of A

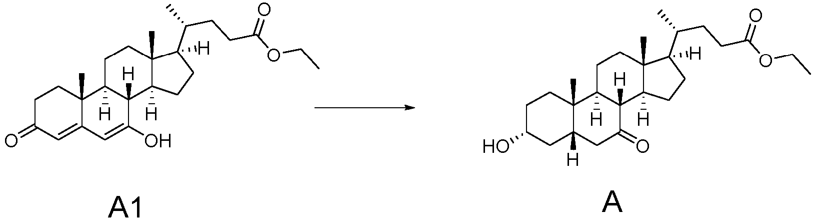

A1 (3.2 g, 7.73 mmol) prepared in Example 17 was dissolved in N,N-dimethylformamide (32 mL) and then 0.32 g of 5% palladium carbon catalyst was added. The reaction mixture was hydrogenated at 25 °C under 0.1 MPa hydrogen for 12 h. After the hydrogenation was completed, the reaction solution was filtered and concentrated under reduced pressure. The crude product was purified by column chromatography (n-hexane:EtOAc = 3:1) to obtain 2.09 g of a white solid A with the yield of 65% and the purity of 91% by HPLC. ESI-MS [M+H]⁺ 419.30.

### Example 22 Synthesis of A

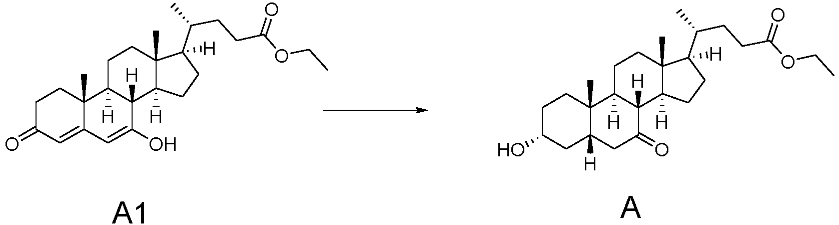

A1 (3.2 g, 7.73 mmol) prepared in Example 17 was dissolved in N,N-dimethylformamide (32 mL) and then 0.32 g of 5% platinum carbon catalyst was added. The reaction mixture was hydrogenated at 25 °C under 0.1 MPa hydrogen for 12 h. After the hydrogenation was completed, the reaction solution was concentrated under reduced pressure. The crude product was purified by column chromatography (n-hexane:EtOAc = 3:1) to obtain 1.70 g of a white solid A with the yield of 53% and the purity of 92% by HPLC. ESI-MS [M+H]⁺ 419.30.

### Example 23 Synthesis of A1-1

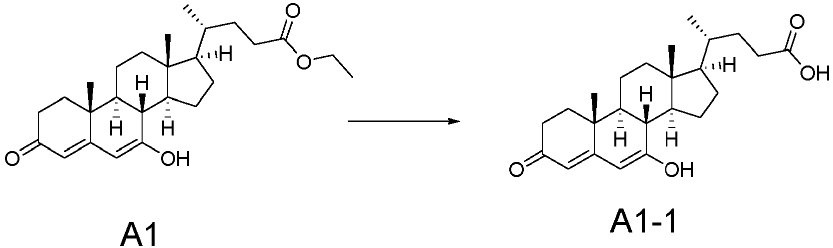

A1 (5 g, 12.08 mmol) prepared in Example 17 was dissolved in THF (20 mL) and water (10 mL) and then 3 N of a NaOH aqueous solution (6 mL, 18mmol) was added. The reaction mixture was reacted at 25 °C for 1 h. When TLC detected that the raw materials were reacted completely, the reaction mixture was cooled to an internal temperature of 0 °C, and 3 N of a HCl aqueous solution (8 mL, 24 mmol) was dropwise added. The reaction solution was extracted with ethyl acetate (30 mL×2). The organic phases were combined, washed with water (30 mL), a saturated sodium chloride solution (30 mL), dried over anhydrous sodium sulfate, and filtered. The filtrate was concentrated under reduced pressure and refined with acetone to obtain 4.38 g of a white solid A1-1 with the yield of 94% and the purity of 98% by HPLC. ESI-MS [M+H]⁺ 387.30.

¹H-NMR(CDCl, 400MHz) δ(ppm): 5.72-5.68(m, 1H), 3.49-3.41(m, 1H), 3.10-3.02(m, 1H), 2.61-2.11(m, 7H), 2.10-1.63(m, 9H), 1.49-1.22(m, 5H), 1.23-1.02(m, 3H), 0.72-0.71(m, 3H).

### Example 24 Synthesis of 7-LCA

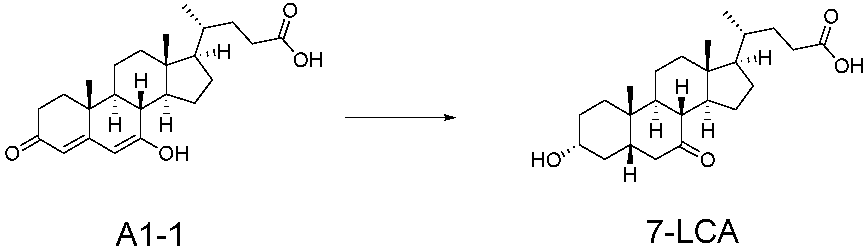

A1-1 (3 g, 7.77 mmol) prepared in Example 23 was dissolved in N,N-dimethylformamide (DMF) (30 mL) and then 0.3 g of Raney nickel was added. The reaction mixture was hydrogenated at 25 °C under 0.1 MPa hydrogen for 12 h. After the hydrogenation was completed, the reaction solution was filtered, concentrated under reduced pressure and refined with acetone to obtain 2.5 g of a white solid 7-LCA with the yield of 82% and the purity of 92% by HPLC. ESI-MS [M+H]⁺ 391.30.

The foregoing exemplarily illustrates the implementation of the technical solutions of the present invention. It should be understood that the protection scope of the present invention is not limited to the above embodiments. Any modifications, equivalent replacements, improvements, and the like made by a person skilled in the art within the spirit and principle of the present invention shall fall within the protection scope of the claims of the present application.

## Claims

1. A method for preparing a compound of formula I, comprising subjecting a compound I-1 to a hydrogenation reaction in the presence of a catalyst to obtain the compound of formula I, 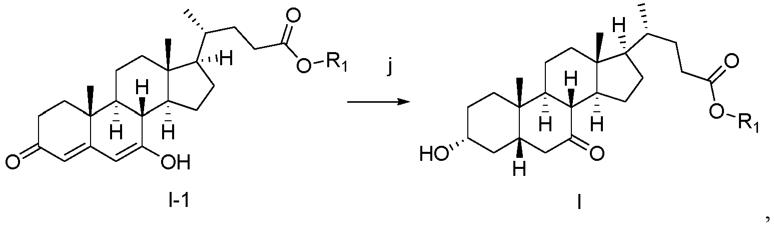 wherein R₁ is H or alkyl, for example, C₁₋₆ alkyl, such as methyl, ethyl, n-propyl or tert-butyl.

2. The method according to claim 1, wherein the catalyst is selected from a Raney Ni catalyst, a Pd/C catalyst, a Pt/C catalyst or a Ru/C catalyst,
preferably, the reaction is performed in the presence of a solvent, wherein the solvent is selected from an amide solvent, for example, the solvent is selected from at least one of N,N-dimethylformamide, N,N-dimethylacetamide, formamide, N-methylpyrrolidone, N-methylformamide, N-methylacetamide and N,N-dimethylpropyleneurea.

3. The method according to claim 1 or 2, wherein when R₁ in the compound of formula I is alkyl, the method further comprises: hydrolyzing the compound of formula I in which R₁ is alkyl to obtain the compound of formula I with R₁ as H,

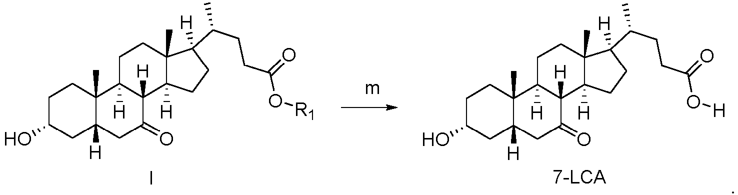

4. An intermediate compound I-1 or I-2 shown below: 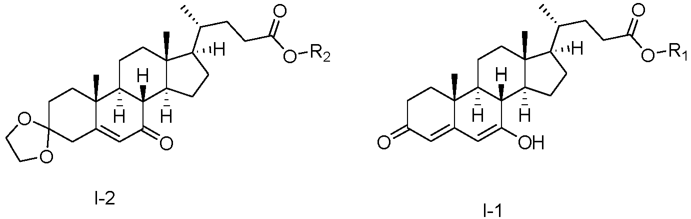 wherein R₁ is defined in claim 1 and R₂ is alkyl.

5. A method for preparing a compound I-1, comprising: 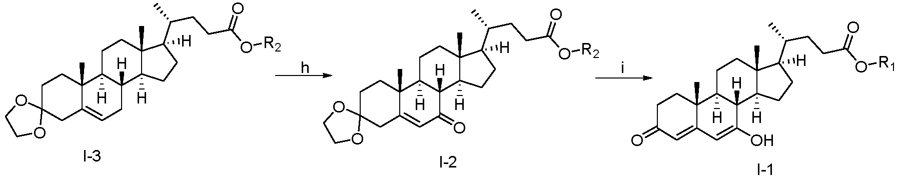 wherein R₁ is defined in claim 1 and R₂ is alkyl,
h) subjecting a compound I-3 to an allylic oxidation reaction to obtain a compound I-2; and
i) subjecting the compound I-2 to a de-protection of ethylene glycol to obtain the compound I-1 with R₁ as alkyl; or subjecting the compound I-2 to a de-protection of ethylene glycol and a hydrolysis reaction to obtain the compound I-1 with R₁ as H.

6. The method according to claim 5, wherein in step h), the reaction is performed in the presence of an oxidant and a catalyst, preferably, step h) is performed under the following reaction system of h1 or h2, wherein
h1: the oxidant is oxygen or air, and the catalyst is a reaction system of N-hydroxyphthalimide (NHPI) and cobalt acetate; and a free radical initiator, for example, benzoyl peroxide, may further be added to the above system; and
h2: the oxidant is tert-butyl hydroperoxide (TBHP) and the catalyst is a reaction system of manganese (III) acetate, manganese (III) acetate dihydrate or cuprous iodide (CuI).

7. The method according to claim 5, wherein in step i), the de-protection of ethylene glycol is performed in the presence of an acid, wherein the acid is selected from at least one of concentrated sulfuric acid, concentrated hydrochloric acid and p-toluenesulfonic acid; and the hydrolysis reaction is performed under an alkaline condition, for example in the presence of sodium hydroxide and/or potassium hydroxide.

8. The method according to claim 5, wherein a method for preparing the compound I-3 comprises the following Method I or Method II, wherein
Method I comprises: 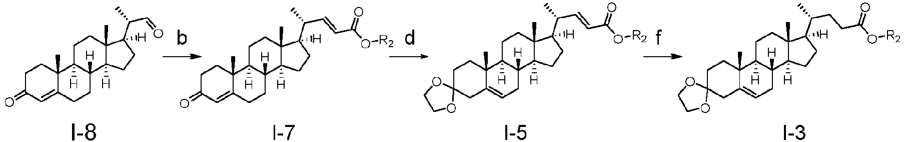 wherein R₂ is defined in claim 5;
b) subjecting a compound I-8 and monoalkyl malonate ( 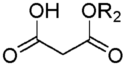 ) to a Knoevenagel condensation to obtain a compound I-7; or subjecting the compound I-8 and 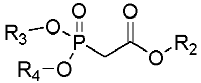 wherein R₃ and R₄ are C₁₋₆ alkyl to a wittig reaction to obtain the compound I-7;
d) subjecting the compound I-7 to a protection of ethylene glycol to obtain a compound I-5; and
f) subjecting the compound I-5 to a reduction reaction to obtain the compound I-3; and
Method II comprises: 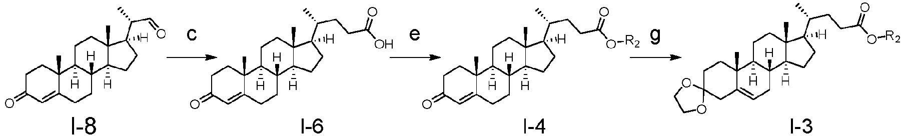 wherein R₂ is defined in claim 5;
c) subjecting the compound I-8 and 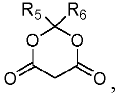 wherein R₅ and R₆ are C₁₋₆ alkyl, or R₅ and R₆ together with the carbon atom to which they are connected form a C₃₋₈ cycloalkyl, to a Knoevenagel condensation to obtain a compound I-6;
e) reacting the compound I-6 with R₂OH to obtain a compound I-4; and
g) subjecting the compound I-4 to a protection of ethylene glycol to obtain a compound I-3.

9. The method according to claim 8, wherein the Knoevenagel condensation of step b) is performed in the presence of a catalyst, wherein the catalyst is DMAP;
step d) is performed in the presence of a catalyst, wherein the catalyst is a catalyst A and/or a catalyst B, wherein the catalyst A is selected from p-toluenesulfonic acid or concentrated sulfuric acid, and the catalyst B is selected from trimethyl orthoformate, triethyl orthoformate or trimethyl orthoacetate; and
step f) is performed in the presence of a catalyst, wherein the catalyst is Pd/C.

10. The method according to claim 8, wherein step e) is performed in the presence of a catalyst, wherein the catalyst is selected from at least one of concentrated sulfuric acid, p-toluenesulfonic acid and hydrochloric acid; and
step g) is performed in the presence of a catalyst, wherein the catalyst is a catalyst A and/or a catalyst B, wherein the catalyst A is selected from p-toluenesulfonic acid or concentrated sulfuric acid, and the catalyst B is selected from trimethyl orthoformate, triethyl orthoformate or trimethyl orthoacetate.
